# FASCICULE DE BREVET EUROPEEN

(11) **EP 4 615 382 B1**
(45) Date de publication et mention de la délivrance du brevet: **11.11.2026**
(21) Numéro de dépôt: 23792949.2
(22) Date de dépôt: 18.10.2023
(51) Int. Cl.: A61F 11/00, A46B 13/02

(54) **NETTOYEUR D'OREILLE**
OHRREINIGER
EAR CLEANER

(30) Priorité: 07.11.2022 CH 1324202022
(43) Date de publication de la demande: 17.09.2025
(73) Titulaire: Oscimed SA, 2300 La Chaux-de-Fonds (CH)
(72) Inventeur: MAGNIN, Jacques, 2300 La Chaux-de-Fonds (CH); CHOLLET, Pascal, 2300 La Chaux-de-Fonds (CH)
(74) Mandataire: Bovard SA Neuchâtel
(86) Numéro de dépôt international: PCT/EP2023/078923
(87) Numéro de publication internationale: WO 2024/099713

(56) Documents cités:
- WO-A1-2015/009839
- US-A1- 2010 312 198
- US-A1- 2021 330 138
- US-B1- 9 549 854

## Description

### Domaine technique

La présente invention concerne un nettoyeur d'oreille comprenant un manche de forme générale de révolution autour d'un axe de révolution définissant l'axe de rotation lors du nettoyage de l'oreille et, à au moins une extrémité du manche, un embout de nettoyage coaxial au manche.

### Etat de la technique

Pour nettoyer les oreilles, il est courant d'employer des cotons-tiges, bâtonnets recouverts à chaque extrémité d'une petite boule de coton. Les cotons-tiges présentent toutefois l'inconvénient de repousser le cérumen plus loin dans le conduit auditif. Cela peut, à terme, créer des bouchons.

De plus, les cotons-tiges jetables doivent être remplacés par des produits plus respectueux de l'environnement, tout en restant efficaces pour le nettoyage des oreilles.

Dans ce but, des cotons-tiges en matières plastiques lavables ont été développés. Mais ils présentent toujours le même inconvénient de repousser le cérumen.

Différentes variantes de nettoyeurs d'oreille sont également proposées. Les nettoyeurs d'oreilles sont moins invasifs qu'un coton-tige, et peuvent généralement être nettoyés pour pouvoir être réutilisés. Il existe par exemple des nettoyeurs d'oreille avec un embout à rainures en spirale, ou un embout en forme de croix formant une spirale dans le conduit auditif durant la rotation. Ces embouts sont réalisés en silicone souple et flexible pour le confort de l'oreille. Ils présentent cependant l'inconvénient de ne pas avoir une action mécanique suffisamment efficace, l'embout étant trop mou.

Il est également proposé des nettoyeurs d'oreille métalliques présentant des têtes de différentes formes : fil métallique enroulé en spirale, cuillère, anneaux... Toutefois, les utilisateurs peuvent avoir une certaine appréhension à introduire une tige métallique dans leurs conduits auditifs.

Les cotons-tiges ou nettoyeurs d'oreille disponibles sur le marché ne sont donc pas satisfaisants.

On connait par exemple le brevet US 9 549 854 qui décrit un nettoyeur d'oreille comprenant un manche de forme générale de révolution autour d'un axe de révolution définissant l'axe de rotation lors du nettoyage de l'oreille et formé par l'intersection d'un plan frontal et d'un plan médian, et, à au moins une extrémité du manche, un embout de nettoyage coaxial au manche.

On connait également les documents WO 2015/009839, US 2010/312198 et US 2021/330138 qui décrivent différents modèles de dispositif de nettoyage du conduit auditif destinés à être insérés dans ledit conduit auditif.

Toutefois, ces dispositifs connus ne permettent pas un nettoyage correct tout en préservant la sécurité du conduit auditif.

Il est donc nécessaire de pouvoir proposer un nettoyeur d'oreille plus performant, permettant une extraction efficace du cérumen tout en préservant la sécurité du conduit auditif.

### Divulgation de l'invention

A cet effet, l'invention concerne un nettoyeur d'oreille comprenant un manche de forme générale de révolution autour d'un axe de révolution définissant l'axe de rotation lors du nettoyage de l'oreille et formé par l'intersection d'un plan frontal et d'un plan médian, et, à au moins une extrémité du manche, un embout de nettoyage coaxial au manche.

Selon l'invention, ledit embout comprend un corps cylindrique coaxial au manche, présentant sur une partie distale une tête comprenant une série d'ailettes disposées radialement sur le pourtour extérieur de la partie distale du corps, chaque ailette s'étendant le long de ladite partie distale suivant l'axe de révolution et présentant une section transversale qui augmente progressivement à partir de l'extrémité distale de la tête conférant à ladite tête une forme ogivale, ledit embout comprenant également une pelle prévue à l'extrémité distale de la tête, dans la continuité de la tête, ladite pelle présentant au moins une face fonctionnelle sensiblement parallèle au plan frontal.

D'une manière avantageuse, la pelle présente une section transversale qui augmente progressivement à partir de son extrémité distale jusqu'à l'extrémité distale de la tête.

De préférence, chaque ailette présente une section transversale augmentant progressivement à partir de l'extrémité distale de la tête puis diminuant progressivement jusqu'à l'extrémité proximale de la tête.

De préférence, le corps de l'embout présente un diamètre extérieur qui augmente progressivement à partir de l'extrémité distale de la partie distale du corps conférant une forme conique.

Ainsi, le nettoyeur d'oreille selon l'invention est d'une grande efficacité, en permettant de capturer le cérumen sans le repousser dans le conduit auditif grâce notamment à la forme progressive conique des ailettes donnant à la tête une forme ogivale, à la forme progressive conique à l'extrémité du corps de l'embout, ainsi qu'à la section réduite de la pelle intégrée à l'extrémité de la tête.

### Brève description des dessins

D'autres caractéristiques et avantages de la présente invention apparaîtront à la lecture de la description détaillée suivante d'un mode de réalisation de l'invention, donnés à titre d'exemple non limitatif, et faite en référence aux dessins annexés dans lesquels :
- la figure 1 est une vue de dessus d'un nettoyeur d'oreille selon l'invention;
- la figure 2 est une vue isométrique de côté du nettoyeur d'oreille de la figure 1, les différents embouts étant séparés du manche ;
- la figure 3 est une vue isométrique de dessus du nettoyeur d'oreille de la figure 1, les différents embouts étant séparés du manche;
- la figure 4 est une vue en coupe selon le plan médian du nettoyeur d'oreille de la figure 1 ;
- la figure 5 est une vue isométrique d'un embout utilisé dans le nettoyeur d'oreille de l'invention ;
- la figure 6 est une vue en coupe selon le plan médian de l'embout de la figure 5 ;
- la figure 7 est une vue en coupe transversale de l'embout de la figure 5 ; et
- la figure 8 est une vue de face de l'embout.

### Modes de réalisation de l'invention

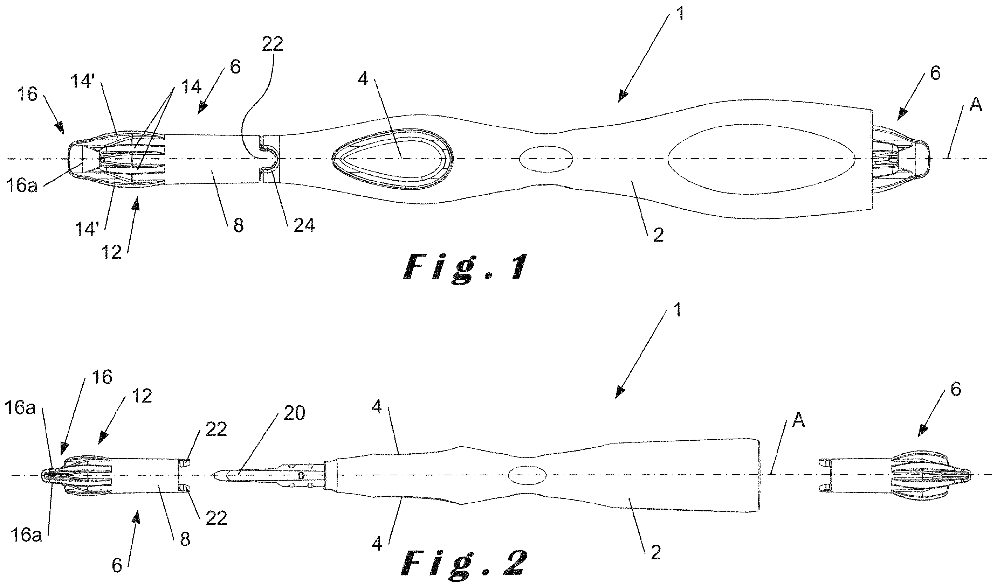

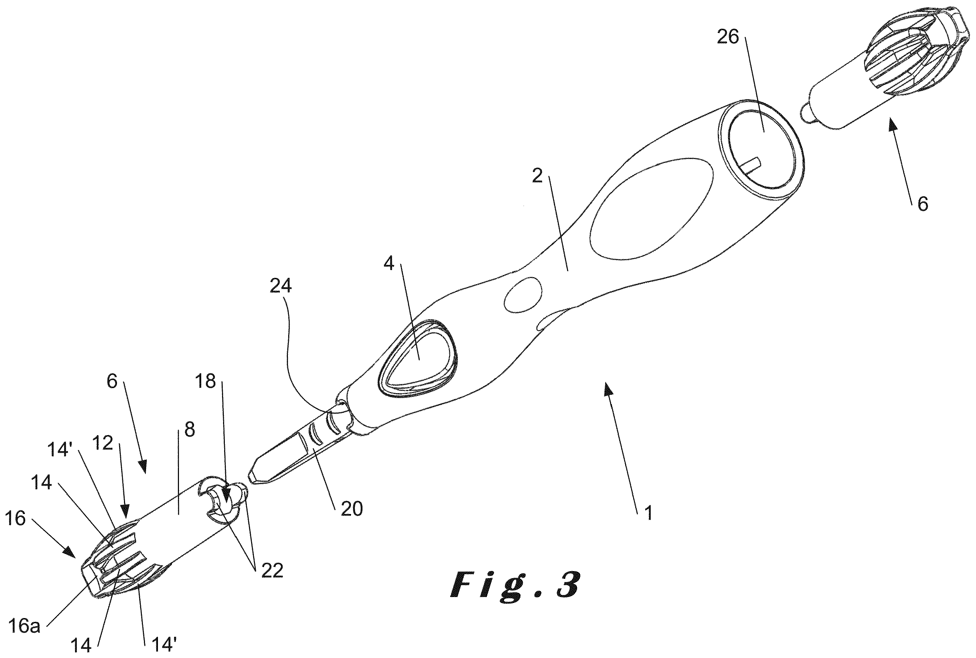

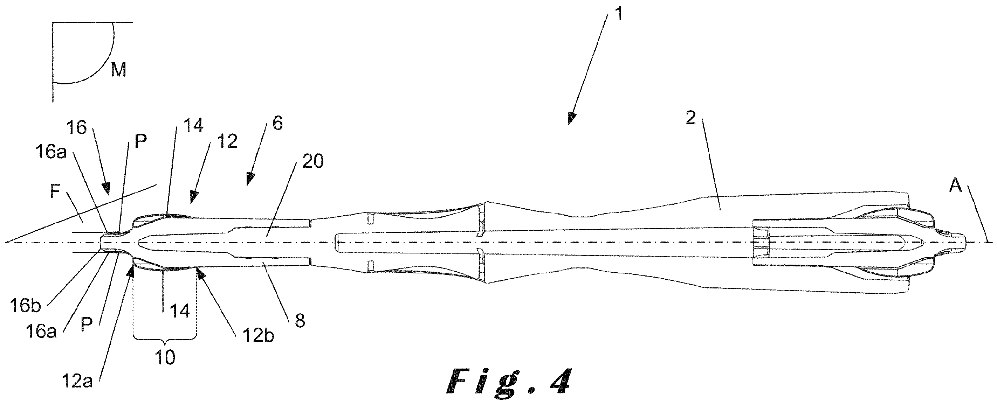

En référence aux figures 1 à 4, la présente invention concerne un nettoyeur d'oreille 1 comprenant un manche 2 de forme générale de révolution autour d'un axe longitudinal de révolution A définissant l'axe de rotation lors du nettoyage de l'oreille. L'axe A est formé par l'intersection d'un plan frontal F et d'un plan médian M, représentés sur la figure 4.

Le diamètre du manche 2 peut varier afin de lui donner une forme ergonomique.

De plus, il est prévu deux méplats 4 parallèles au plan frontal F dont le rôle sera décrit plus en détails ci-après.

Le nettoyeur d'oreille 1 comprend également, à au moins une extrémité du manche 2, un embout 6 de nettoyage coaxial au manche 2, agencé pour pouvoir être assemblé de manière solidaire au manche 2.

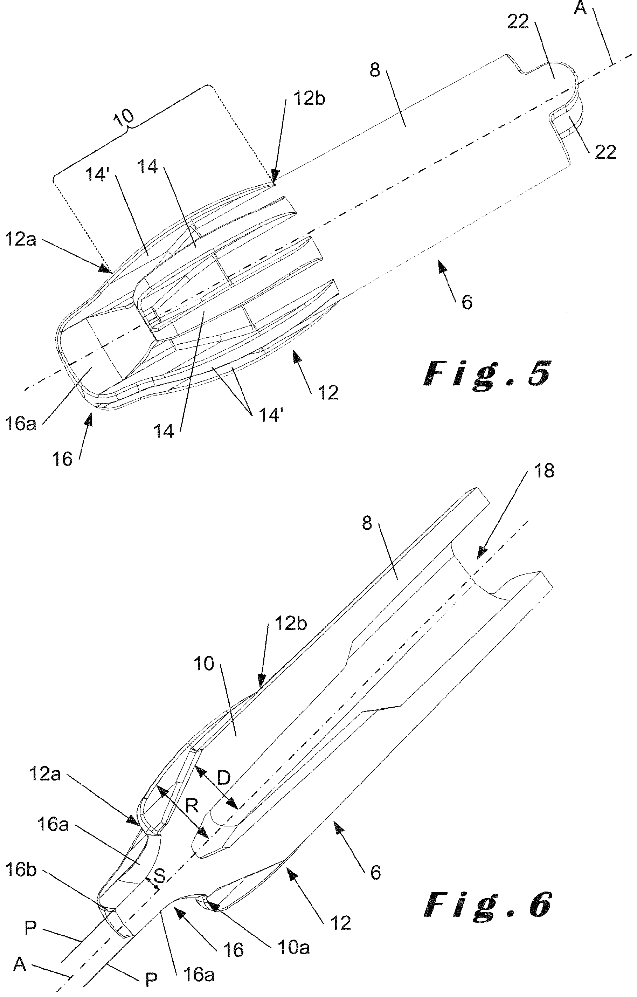

Selon l'invention, et en référence plus particulièrement aux figures 5 à 8, ledit embout 6 comprend un corps cylindrique 8 coaxial au manche 2, agencé pour pouvoir être assemblé de manière solidaire au manche 2, et présentant sur une partie distale 10 (i.e. à l'extrémité du corps 8 opposée au manche 2) une tête 12 comprenant une série d'ailettes 14 pleines, disposées radialement et régulièrement réparties sur le pourtour extérieur de la partie distale 10 du corps 8, chaque ailette 14 s'étendant le long de ladite partie distale 10 suivant l'axe de révolution A. La tête 12 a ainsi une fonction rotative, chaque ailette 14 permettant d'extraire le cérumen en tournant sur les parois du conduit auditif.

Dans l'exemple représenté, l'embout comprend huit ailettes 14, mais il est bien évident que le nombre d'ailettes peut varier.

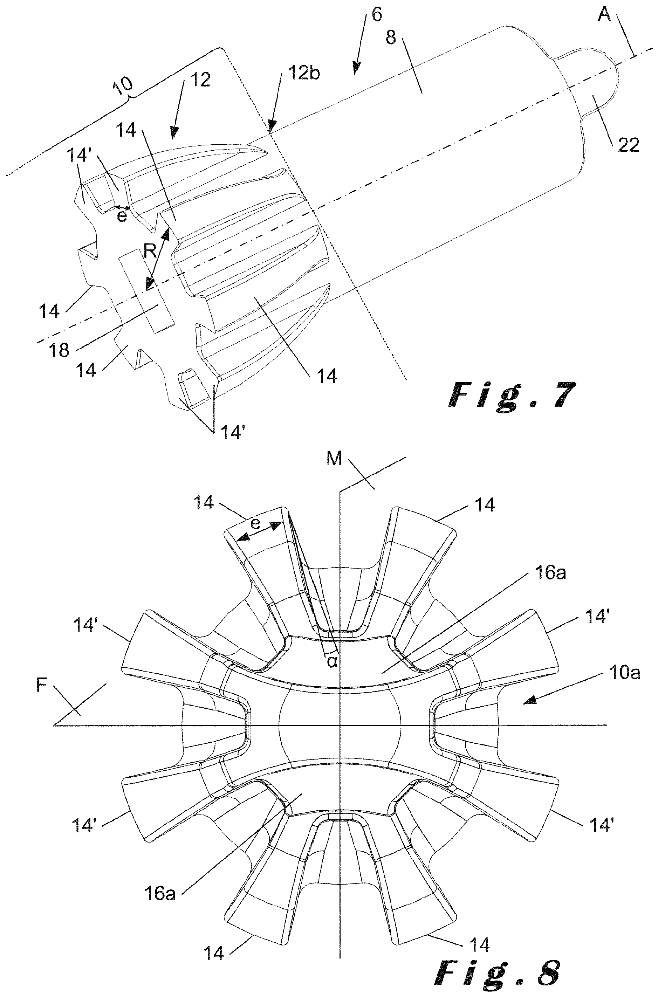

D'une manière avantageuse, chaque ailette 14 présente une section transversale (i.e. dans un plan perpendiculaire à l'axe A) qui augmente progressivement à partir de l'extrémité distale 12a de la tête 12 conférant à ladite tête 12 une forme ogivale. Ainsi, le rayon extérieur R (cf. figures 6 et 7) de chaque ailette 14, définissant sa hauteur vue en coupe transversale dans un plan radial, augmente progressivement à partir de l'extrémité distale 12a de la tête 12. Cela permet de ne pas repousser le cérumen et de faciliter l'insertion de l'embout dans le conduit auditif.

En outre, ledit embout 6 comprend une pelle 16 prévue à l'extrémité distale 12a de la tête 12, dans la continuité de ladite tête 12, ladite pelle 16 présentant au moins une face fonctionnelle 16a s'étendant dans un plan P sensiblement parallèle au plan frontal F (cf. figures 4 et 6). Cela signifie que la face fonctionnelle 16a peut être plane, parallèle au plan frontal F ou qu'elle peut être légèrement incurvée pour former, en vue de face, une section transversale de forme concave. L'angle de courbure de la face fonctionnelle 16a par rapport au plan frontal F peut être par exemple compris entre 0° (face fonctionnelle 16a plane, parallèle au plan frontal F ) et 30° (face fonctionnelle 16a incurvée), de préférence compris entre 10° et 25°.

De préférence, du fait de la symétrie de l'embout 6, la pelle 16 comprend deux faces fonctionnelles 16a s'étendant de part et d'autre du plan frontal F.

D'une manière avantageuse, la pelle 16 présente, de part et d'autre du plan frontal F, en vue de face, une section transversale de forme concave, de préférence symétrique par rapport audit plan frontal F, donnant à ladite pelle 16 un aspect extérieur biconcave comme cela apparait sur la figure 8. Cette forme biconcave de la pelle 16, avec un angle de courbure par rapport au plan frontal F inférieur à 30°, et de préférence compris entre 10° et 25°, permet très avantageusement de réduire l'impact dans le cérumen et d'avoir un meilleur effet raclant. Il est bien évident que, dans une autre variante non représentée la pelle pourrait présenter une face fonctionnelle incurvée et une face fonctionnelle plane, ou de courbure différente.

D'une manière avantageuse, la pelle 16 présente une section transversale S (cf. figure 6) qui augmente progressivement à partir de son extrémité distale 16a jusqu'à l'extrémité distale 12a de la tête 12. Ainsi, l'extrémité distale 16a de la pelle 16 est de section la plus fine possible pour rejoindre l'extrémité de la tête 12 en pente douce. La faible section de la pelle 16 permet d'extraire le cérumen en raclant le fond de l'oreille sans repousser le cérumen au fond du conduit auditif.

De préférence, chaque ailette 14 présente, en suivant l'axe A, une section transversale (i.e. définie par le rayon extérieur R en coupe transversale dans un plan radial) augmentant progressivement à partir de l'extrémité distale 12a de la tête 12 puis diminuant progressivement jusqu'à l'extrémité proximale 12b de la tête 12 (cf. figures 6 et 7). Cela signifie que chaque ailette 14 présente une hauteur, prise depuis la base de l'ailette 14 dans un plan radial, qui augmente progressivement à partir de l'extrémité distale 12a de la tête 12 puis qui diminue progressivement jusqu'à l'extrémité proximale 12b de la tête 12 en suivant l'axe A. Ceci permet d'éviter les arêtes vives pour le confort de l'utilisateur.

De préférence, chaque ailette 14 présente un angle de coupe α positif, par exemple compris entre 0.5° et 20°.

De plus, chaque ailette 14 présente, dans un plan transversal, une forme s'évasant en direction de l'extérieur, chaque ailette 14 présentant une épaisseur e qui augmente progressivement depuis la base de l'ailette 14 (reliant la partie distale 10 du corps 8) en direction de l'extérieur dans un même plan transversal (cf. figures 7 et 8). L'ailette 14, prise dans un plan transversal, peut s'évaser selon un angle compris entre 0.5° et 45° par rapport à l'axe de symétrie de l'ailette 14 dans le plan transversal. Cela permet un meilleur effet raclant et non repoussant.

D'une manière avantageuse, la tête 12 comprend, d'un même côté du plan frontal F, et de chaque côté dudit plan frontal F en symétrie par rapport audit plan frontal F, deux ailettes latérales, référencées plus spécifiquement 14' sur les figures, qui se prolongent au-delà de l'extrémité distale 12a de la tête 12 pour se rejoindre donnant à la pelle 16 son aspect extérieur biconcave et en formant au centre l'une des faces fonctionnelles 16a incurvée de la pelle 16. Les autres caractéristiques décrites ci-dessus d'une manière générale pour les ailettes 14 s'appliquent également aux ailettes référencées 14'.

De préférence, le corps 8 de l'embout 6 présente un diamètre extérieur D qui augmente progressivement à partir de l'extrémité distale 10a de la partie distale 10 du corps 8 conférant une forme conique à l'extrémité distale du corps 8 qui s'évase à partir de la pelle 16. En d'autres termes, le diamètre extérieur D de l'extrémité de la partie distale 10 du côté de la pelle 16 diminue progressivement en direction de ladite pelle 16. La forme conique de l'extrémité distale du corps 8 à partir de la pelle 16 permet de ne pas repousser le cérumen au fond du conduit auditif.

De préférence, l'embout 6, c'est-à-dire le corps 8, les ailettes 14 et la tête 16, est réalisé dans un seul matériau, par exemple par moulage. Un tel matériau peut être par exemple un silicone, un EVA (éthyle vinyle acétate), un TPE (élastomère thermoplastique) ou un PU (polyuréthane), ou tout autre matériau approprié.

D'une manière avantageuse, le corps 8 de l'embout 6 comprend une âme creuse 18 (cf. figure 6) agencée pour recevoir une armature longitudinale 20 prévue à une extrémité du manche 2 afin de monter l'embout 6 sur le manche 2 en insérant l'armature 20 dans l'âme 18. La forme de l'armature 20 correspond à la forme de l'âme 18 afin d'assurer un bon maintien de l'embout 6 sur le manche 2.

De préférence, l'armature 20 s'étend approximativement jusqu'à l'extrémité distale 10a de la partie distale 10 du corps 8. L'armature 20 est avantageusement réalisée dans un matériau plus rigide que le corps 8 afin de constituer un squelette rigide pour l'embout 6 permettant une meilleure insertion dans le conduit auditif. Le manche 2 et son armature 20 peuvent être réalisés par exemple en POM (polyoxyméthylène), PET (polyéthylène téréphtalate), PP (polypropylène), PA (polyamide), ou tout autre matériau approprié.

D'une manière avantageuse, l'embout 6 est agencé pour être monté sur le manche 2 de manière amovible afin de pouvoir être remplacé par un embout de taille différente ou par un nouvel embout 6.

Afin d'assurer le verrouillage de l'embout 6 sur le manche 2, il peut être par exemple prévu à l'extrémité proximale du corps 8 des moyens de verrouillage, tels que des pattes 22, agencés pour s'engager dans des encoches 24 correspondantes prévues sur le manche 2.

D'une manière avantageuse, l'embout 6 et le manche 2 comprennent des moyens d'indexage agencés pour assembler l'embout 6 et le manche 2 de telle sorte que le plan principal P des faces fonctionnelles 16a de la pelle 16 est parallèle au plan frontal F, une fois l'embout 6 monté sur le manche 2.

Dans un mode de réalisation préféré, les moyens de verrouillage, tels que les pattes 22, et les encoches correspondantes 24, sont positionnés respectivement sur le pourtour de l'embout 6 et le pourtour du manche 2 de manière à constituer également les moyens d'indexage. Ainsi, l'embout 6 est automatiquement positionné correctement par rapport au manche 2 lors de l'assemblage.

De ce fait, les méplats 4 parallèles au plan frontal F sont également parallèles au plan principal P des faces fonctionnelles 16a de la pelle 16, une fois l'embout 6 monté sur le manche 2. Ainsi, le manche 2 comprend une zone de préhension cylindrique, constituée par la partie cylindrique du manche 2, et une zone de préhension plate parallèle au plan frontal F et au plan principal P des faces fonctionnelles de la pelle, ladite zone de préhensions plate étant constituée par les méplats 4.

La zone de préhension cylindrique permet une prise en main avec les doigts pour facilement faire tourner le manche 2 lors de l'utilisation des ailettes 14 pour un nettoyage rotatif. La zone de préhension plate permet une prise en main avec les doigts afin de garantir la bonne orientation des faces fonctionnelles de la pelle 16 lors de son utilisation.

Il est bien évident que, dans un autre mode de réalisation, l'embout 6 peut être monté de manière non amovible sur le manche 2.

La longueur du corps 8 de l'embout 6 est choisie de manière à correspondre à une longueur de sécurité pour éviter de perdre l'embout 6 dans l'oreille en cas de casse accidentelle du manche ou en cas de déverrouillage accidentel.

D'une manière avantageuse, le manche 2 comprend à une extrémité un embout de nettoyage 6 et à l'autre extrémité un logement intérieur 26 agencé pour recevoir au moins un autre embout de nettoyage 6. Les deux embouts de nettoyage 6 peuvent être de même taille ou de taille différente. Dans l'exemple représenté, le logement intérieur 26 est dimensionné pour laisser apparaitre notamment la pelle 16 et une partie de la tête 12 de l'embout 6. La base dudit logement intérieur 26 présente un socle sur lequel le corps 8 de l'embout 6 vient se clipser, assurant le maintien de l'embout 6 dans le logement 26. Dans une autre variante non représentée, le logement intérieur 26 peut être dimensionné pour pouvoir y loger entièrement un embout 6, ledit logement 26 étant alors fermé par un bouchon (non représenté).

De préférence, le nettoyeur d'oreille 1 est proposé avec deux embouts 6 de taille différente.

Le nettoyeur d'oreille 1 selon l'invention a une double fonction d'extraction et de raclage grâce aux ailettes 14 et à la pelle 16. La fonction rotative des ailettes 14 permet d'extraire le cérumen en tournant, sans déformation en spirale, sur les parois du conduit auditif via la rotation de la zone de préhension cylindrique du manche 2. La pelle 16, correctement orientée grâce à la zone de préhension plate, permet de racler le fond de l'oreille. La pelle 16 est auto-guidée grâce à la forme ogivale de la tête12 qui permet de créer un effet de rotule et de centrage pour ladite pelle 16.

La forme progressive conique des ailettes 14 conférant à la tête 12 une forme ogivale d'insertion, la forme progressive conique de l'extrémité distale du corps 8 de l'embout 6, ainsi qu'à la section réduite de la pelle 16 permettent de capturer le cérumen et de l'extraire sans le repousser dans le conduit auditif.

Il est bien évident que, selon une autre réalisation possible, le nettoyeur d'oreille peut comprendre un manche 2 de forme générale de révolution autour d'un axe de révolution A définissant l'axe de rotation lors du nettoyage de l'oreille et formé par l'intersection d'un plan frontal F et d'un plan médian M, et, à au moins une extrémité du manche 2, un embout 6 de nettoyage coaxial au manche 2, dans lequel ledit embout 6 comprend un corps cylindrique 8 coaxial au manche 2 présentant sur une partie distale 10 une tête 12 présentant une configuration appropriée, différente des ailettes décrites ci-dessus, et dans lequel ledit embout 6 comprend une pelle 16 prévue à l'extrémité distale 12a de la tête 12, dans la continuité de ladite tête 12, ladite pelle 16 présentant au moins une face fonctionnelle 16a sensiblement parallèle au plan frontal F, c'est-à-dire plane ou incurvée, comme décrit ci-dessus. De préférence, la pelle 16 présente, de part et d'autre du plan frontal F, en vue de face, une section transversale de forme concave, de préférence symétrique par rapport audit plan frontal F, donnant à ladite pelle 16 un aspect extérieur biconcave comme cela apparait sur la figure 8. Toutes les autres caractéristiques décrites ci-dessus peuvent être combinées à ce mode de réalisation.

Le nettoyeur d'oreille 1 selon l'invention est réutilisable, les embouts 6 pouvant être facilement être retirés pour être nettoyés.

## Revendications

1. Nettoyeur d'oreille (1) comprenant un manche (2) de forme générale de révolution autour d'un axe de révolution (A) définissant l'axe de rotation lors du nettoyage de l'oreille et formé par l'intersection d'un plan frontal (F) et d'un plan médian (M), et, à au moins une extrémité du manche (2), un embout (6) de nettoyage coaxial au manche (2), **caractérisé en ce que** ledit embout (6) comprend un corps cylindrique (8) coaxial au manche (2) présentant sur une partie distale (10) une tête (12) comprenant une série d'ailettes (14) disposées radialement sur le pourtour extérieur de ladite partie distale (10) du corps (8), chaque ailette (14) s'étendant le long de ladite partie distale (10) suivant l'axe de révolution (A) et présentant une section transversale qui augmente progressivement à partir de l'extrémité distale (12a) de la tête (12) conférant à la tête (12) une forme ogivale, et **en ce que** ledit embout (6) comprend une pelle (16) prévue à l'extrémité distale (12a) de la tête (12), dans la continuité de ladite tête (12), ladite pelle (16) présentant au moins une face fonctionnelle (16a) sensiblement parallèle au plan frontal (F).

2. Nettoyeur d'oreille (1) selon la revendication 1, **caractérisé en ce que** la pelle (16) présente une section transversale qui augmente progressivement à partir de son extrémité distale (16b) jusqu'à l'extrémité distale (12a) de la tête (12).

3. Nettoyeur d'oreille (1) selon l'une des revendications précédentes, **caractérisé en ce que** chaque ailette (14) présente une section transversale augmentant progressivement à partir de l'extrémité distale (12a) de la tête (12) puis diminuant progressivement jusqu'à l'extrémité proximale (12b) de la tête (12).

4. Nettoyeur d'oreille (1) selon l'une des revendications précédentes, **caractérisé en ce que** chaque ailette (14) présente un angle de coupe (α) positif.

5. Nettoyeur d'oreille (1) selon l'une des revendications précédentes, **caractérisé en ce que** chaque ailette (14) présente, dans un plan transversal, une forme s'évasant en direction de l'extérieur.

6. Nettoyeur d'oreille (1) selon l'une des revendications précédentes, **caractérisé en ce que** le corps (8) de l'embout (8) présente un diamètre extérieur (D) qui augmente progressivement à partir de l'extrémité distale (10a) de la partie distale (10) du corps (8) conférant une forme conique.

7. Nettoyeur d'oreille (1) selon l'une des revendications précédentes, **caractérisé en ce que** le corps (8) de l'embout (6) comprend une âme creuse (18) agencée pour recevoir une armature (20) prévue à une extrémité du manche (2) afin de monter l'embout (6) sur le manche (2).

8. Nettoyeur d'oreille (1) selon l'une des revendications précédentes, **caractérisé en ce que** l'embout (6) est agencé pour être monté sur le manche (2) de manière amovible.

9. Nettoyeur d'oreille (1) selon l'une des revendications précédentes, **caractérisé en ce que** l'embout (6) et le manche (2) comprennent des moyens d'indexage (22, 24) agencés pour assembler l'embout (6) et le manche (2) de sorte que la face fonctionnelle (16a) de la pelle (16) est parallèle au plan frontal (F).

10. Nettoyeur d'oreille (1) selon l'une des revendications précédentes, **caractérisé en ce que** le corps (8) est réalisé dans un matériau plus rigide que le matériau des ailettes (14) et de la tête (16).

11. Nettoyeur d'oreille (1) selon l'une des revendications précédentes, **caractérisé en ce que** le manche (2) comprend une zone de préhension cylindrique et une zone de préhension plate parallèle au plan frontal (F).

12. Nettoyeur d'oreille (1) selon l'une des revendications précédentes, **caractérisé en ce que** le manche (2) comprend à une extrémité un embout de nettoyage (6) et à l'autre extrémité un logement intérieur (26) agencé pour recevoir au moins un embout de nettoyage (6).

13. Nettoyeur d'oreille (1) selon l'une des revendications précédentes, **caractérisé en ce que** la pelle (16) comprend deux faces fonctionnelles (16a) s'étendant de part et d'autre du plan frontal (F).

14. Nettoyeur d'oreille (1) selon l'une des revendications précédentes, **caractérisé en ce que** la pelle (16) présente, de part et d'autre du plan frontal (F), une section transversale de forme concave, donnant à ladite pelle (16) un aspect biconcave.

15. Nettoyeur d'oreille (1) selon l'une des revendications précédentes, caractérisé ce que la tête (12) comprend, d'un même côté du plan frontal (F), deux ailettes latérales (14') qui se prolongent au-delà de l'extrémité distale (12a) de la tête (12) pour se rejoindre en formant une face fonctionnelle (16a) de la pelle (16).

## Patentansprüche

1. Ohrreiniger (1) umfassend einen Griff (2) von der allgemeinen Form eines Rotationskörpers um eine Rotationsachse (A), die die Drehachse bei der Reinigung des Ohrs definiert und durch den Schnitt einer Frontalebene (F) und einer Medianebene (M) gebildet wird, und, an mindestens einem Ende des Griffs (2), einen zum Griff (2) koaxialen Aufsatz (6) zum Reinigen, **dadurch gekennzeichnet, dass** der besagte Aufsatz (6) einen zum Griff (2) koaxialen zylindrischen Körper (8) umfasst, der auf einem distalen Teil (10) einen Kopf (12) aufweist, der eine Reihe von Lamellen (14) umfasst, die auf dem äusseren Rand des besagten distalen Teils (10) des Körpers (8) radial angeordnet sind, wobei jede Lamelle (14) sich entlang des besagten distalen Teils (10) der Rotationsachse (A) folgend erstreckt und einen Querschnitt aufweist, der ausgehend vom distalen Ende (12a) des Kopfes (12) progressiv zunimmt, wodurch dem Kopf (12) eine spitz zulaufende Form verliehen wird, und dadurch, dass der besagte Aufsatz (6) einen Löffel (16) aufweist, der am distalen Ende (12a) des Kopfes (12) in Fortführung des besagten Kopfes (12) vorgesehen ist, wobei der besagte Löffel (16) mindestens eine Funktionsseite (16a) aufweist, die im Wesentlichen parallel zur Frontalebene (F) ist.

2. Ohrreiniger (1) nach Anspruch 1, **dadurch gekennzeichnet, dass** der Löffel (16) einen Querschnitt aufweist, der ausgehend von seinem distalen Ende (16b) bis zum distalen Ende (12a) des Kopfes (12) progressiv zunimmt.

3. Ohrreiniger (1) nach einem der vorstehenden Ansprüche, **dadurch gekennzeichnet, dass** jede Lamelle (14) einen Querschnitt aufweist, der ausgehend vom distalen Ende (12a) des Kopfes (12) progressiv zunimmt, anschliessend bis zum proximalen Ende (12b) des Kopfes (12) progressiv abnimmt.

4. Ohrreiniger (1) nach einem der vorstehenden Ansprüche, **dadurch gekennzeichnet, dass** jede Lamelle (14) einen positiven Schnittwinkel (α) aufweist.

5. Ohrreiniger (1) nach einem der vorstehenden Ansprüche, **dadurch gekennzeichnet, dass** jede Lamelle (14) in einer transversalen Ebene eine nach aussen hin geschweifte Form aufweist.

6. Ohrreiniger (1) nach einem der vorstehenden Ansprüche, **dadurch gekennzeichnet, dass** der Körper (8) des Aufsatzes (8) einen Aussendurchmesser (D) aufweist, der ausgehend vom distalen Ende (10a) des distalen Teils (10) des Körpers (8) progressiv zunimmt, wodurch eine konische Form verliehen wird.

7. Ohrreiniger (1) nach einem der vorstehenden Ansprüche, **dadurch gekennzeichnet, dass** der Körper (8) des Aufsatzes (6) einen Hohlraum (18) aufweist, der ausgelegt ist, um eine an einem Ende des Griffs (2) vorgesehene Armatur (20) aufzunehmen, um den Aufsatz (6) auf dem Griff (2) zu montieren.

8. Ohrreiniger (1) nach einem der vorstehenden Ansprüche, **dadurch gekennzeichnet, dass** der Aufsatz (6) ausgelegt ist, um unbeweglich auf dem Griff (2) montiert zu werden.

9. Ohrreiniger (1) nach einem der vorstehenden Ansprüche, **dadurch gekennzeichnet, dass** der Aufsatz (6) und der Griff (2) Indexiermittel (22, 24) umfassen, die ausgelegt sind, um den Aufsatz (6) und den Griff (2) so zusammenzusetzen, dass die Funktionsseite (16a) des Löffels (16) parallel zur Frontalebene (F) ist.

10. Ohrreiniger (1) nach einem der vorstehenden Ansprüche, **dadurch gekennzeichnet, dass** der Körper (8) aus einem steiferen Material als das Material der Lamellen (14) und des Kopfes (16) gefertigt ist.

11. Ohrreiniger (1) nach einem der vorstehenden Ansprüche, **dadurch gekennzeichnet, dass** der Griff (2) eine zylindrische Greifzone und eine flache Greifzone parallel zur Frontalebene (F) umfasst.

12. Ohrreiniger (1) nach einem der vorstehenden Ansprüche, **dadurch gekennzeichnet, dass** der Griff (2) an einem Ende einen Aufsatz (6) zum Reinigen und an dem anderen Ende eine innere Aufnahme (26), die ausgelegt ist, um mindestens einen Aufsatz (6) zum Reinigen aufzunehmen, umfasst.

13. Ohrreiniger (1) nach einem der vorstehenden Ansprüche, **dadurch gekennzeichnet, dass** der Löffel (16) zwei Funktionsseiten (16a) umfasst, die sich auf beiden Seiten der Frontalebene (F) erstrecken.

14. Ohrreiniger (1) nach einem der vorstehenden Ansprüche, **dadurch gekennzeichnet, dass** der Löffel (16) auf beiden Seiten der Frontalebene (F) einen Querschnitt von konkaver Form aufweist, der dem besagten Löffel (16) ein bikonkaves Aussehen gibt.

15. Ohrreiniger (1) nach einem der vorstehenden Ansprüche, **dadurch gekennzeichnet, dass** der Kopf (12) auf derselben Seite der Frontalebene (F) zwei seitliche Lamellen (14') umfasst, die sich über das distale Ende (12a) des Kopfes (12) hinaus verlängern, um sich zu verbinden und dabei eine Funktionsseite (16a) des Löffels (16) zu bilden.

## Claims

1. An ear cleaner (1) comprising a handle (2) having a generally rotationally symmetrical shape about an axis of revolution (A) defining the axis of rotation during the cleaning of the ear and formed by the intersection of a frontal plane (F) and a median plane (M), and, at at least one end of the handle (2), a cleaning tip (6) coaxial with the handle (2), **characterised in that** said tip (6) comprises a cylindrical body (8) coaxial with the handle (2) and having, on a distal portion (10), a head (12) comprising a series of fins (14) arranged radially on the outer periphery of said distal portion (10) of the body (8), each fin (14) extending along said distal portion (10) along the axis of revolution (A) and having a cross-section that gradually increases from the distal end (12a) of the head (12), giving the head (12) an arcuate shape, and **in that** said tip (6) comprises a scoop (16) provided at the distal end (12a) of the head (12), in the continuity of said head (12), said scoop (16) having at least one functional surface (16a) which is substantially parallel to the frontal plane (F).

2. The ear cleaner (1) according to Claim 1, **characterised in that** the scoop (16) has a cross-section that gradually increases from its distal end (16b) up to the distal end (12a) of the head (12).

3. The ear cleaner (1) according to any one of the preceding claims, **characterised in that** each fin (14) has a cross-section gradually increasing from the distal end (12a) of the head (12) then gradually reducing up to the proximal end (12b) of the head (12).

4. The ear cleaner (1) according to any one of the preceding claims, **characterised in that** each fin (14) has a positive rake angle (α).

5. The ear cleaner (1) according to any one of the preceding claims, **characterised in that** each fin (14) has, in a transversal plane, a shape widening in the direction of the exterior.

6. The ear cleaner (1) according to any one of the preceding claims, **characterised in that** the body (8) of the tip (8) has an outer diameter (D) that gradually increases from the distal end (10a) of the distal portion (10) of the body (8), giving a conical shape.

7. The ear cleaner (1) according to any one of the preceding claims, characterised it that the body (8) of the tip (6) comprises a hollow core (18) arranged to receive a framework (20) provided at one end of the handle (2) in order to mount the tip (6) on the handle (2).

8. The ear cleaner (1) according to any one of the preceding claims, **characterised in that** the tip (6) is arranged to be mounted on the handle (2) in a removable manner.

9. The ear cleaner (1) according to any one of the preceding claims, **characterised in that** the tip (6) and the handle (2) comprise indexing means (22, 24) arranged to assemble the tip (6) and the handle (2) such that the functional surface (16a) of the scoop (16) is parallel to the frontal plane (F).

10. The ear cleaner (1) according to any one of the preceding claims, **characterised in that** the body (8) is made of a more rigid material than the material of the fins (14) and of the head (16).

11. The ear cleaner (1) according to any one of the preceding claims, **characterised in that** the handle (2) comprises a cylindrical gripping zone and a flat gripping zone parallel to the frontal plane (F).

12. The ear cleaner (1) according to any one of the preceding claims, **characterised in that** the handle (2) comprises, at one end, a cleaning tip (6) and, at the other end, an inner housing (26) arranged to receive at least one cleaning tip (6).

13. The ear cleaner (1) according to any one of the preceding claims, **characterised in that** the scoop (16) comprises two functional surfaces (16a) extending on either side of the frontal plane (F).

14. The ear cleaner (1) according to any one of the preceding claims, **characterised in that** the scoop (16) has, on either side of the frontal plane (F), a concave-shaped cross-section, giving said scoop (16) a biconcave appearance.

15. The ear cleaner (1) according to any one of the preceding claims, **characterised in that** the head (12) comprises, on a same side of the frontal plane (F), two lateral fins (14') that extend beyond the distal end (12a) of the head (12) in order to join one another, forming a functional surface (16a) of the scoop (16).
